(19) Europäisches Patentamt
European Patent Office
Office européen des brevets

(11) **EP 3 030 181 B1**

(12) **EUROPÄISCHE PATENTSCHRIFT**

(45) Veröffentlichungstag und Bekanntmachung des Hinweises auf die Patenterteilung:
**04.01.2017  Patentblatt 2017/01**

(51) Int Cl.:
***A61B 18/12*** *(2006.01)*    *A61B 18/00* *(2006.01)*

(21) Anmeldenummer: **15784689.0**

(86) Internationale Anmeldenummer:
**PCT/EP2015/074596**

(22) Anmeldetag: **23.10.2015**

(87) Internationale Veröffentlichungsnummer:
**WO 2016/066542 (06.05.2016 Gazette 2016/18)**

(54) **VORRICHTUNG ZUR STEUERUNG EINES BEHANDLUNGSVORGANGS**

DEVICE FOR CONTROLLING A TREATMENT PROCESS

DISPOSITIF DE COMMANDE D'UN PROCESSUS DE TRAITEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.10.2014  DE 102014115868**

(43) Veröffentlichungstag der Anmeldung:
**15.06.2016  Patentblatt 2016/24**

(73) Patentinhaber: **Aesculap AG**
**78532 Tuttlingen (DE)**

(72) Erfinder:
• **KELLER, Anton**
**78589 Dürbheim (DE)**
• **EICK, Stefan**
**78532 Tuttlingen (DE)**
• **MASER, Thomas**
**78658 Zimmern ob Rottweil (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
**US-A1- 2010 179 534    US-A1- 2011 160 725
US-A1- 2013 338 665    US-B2- 6 733 498**

**Beschreibung**

Die Erfindung betrifft eine Vorrichtung zur Steuerung eines Behandlungsvorgangs bei, vor oder nach einer chirurgischen oder nichtchirurgischen, oder einer therapeutischen oder nicht-therapeutischen Behandlung eines Materials, beispielsweise eines Gewebes, das nichtmenschlicher oder nichttierischer, gegebenenfalls auch menschlicher oder tierischer Art sein kann.

Aus der US 6,733,498 B2 ist ein Verfahren zum Behandeln von biologischem Gewebe unter Anlegen einer Hochfrequenzspannung an Elektroden eines HF-Chirurgischen Instruments (HF = Hochfrequenz) bekannt, bei dem die Gewebeimpedanz überwacht wird. Während einer ersten Stufe wird ein minimaler Gewebeimpedanzwert detektiert und eine relative Gewebeimpedanz bestimmt. Hierbei wird erfasst, wann die relative Gewebeimpedanz einen vorbestimmten Wert erreicht, und dann auf eine zweite Stufe umgeschaltet, wobei die Dauer der zweiten Stufe als Funktion der Dauer der ersten Stufe berechnet wird. Während der zweiten Stufe wird ebenfalls eine Hochfrequenzspannung an die Elektroden des Behandlungswerkzeugs angelegt.

Aus der EP 2 025 297 A2 ist ein elektrisches Behandlungssystem bekannt, dessen Behandlungswerkzeug während einer ersten Behandlungsphase zur Verklebung von Gewebe mittels Hochfrequenzenergie gespeist wird. In einer zweiten Behandlungsphase wird ein Austrocknungsprozess zur Dehydrierung des behandelten Gewebes durchgeführt. Eine Steuerung schaltet von der ersten Behandlungsphase auf die zweite Behandlungsphase um, wenn ein Phasendifferenzsignal detektiert wird. Hierbei wird das Anliegen der Hochfrequenzenergie beendet, wenn die während der zweiten Behandlungsphase detektierte Phasendifferenz einen vorbestimmten Phasendifferenzwert überschreitet.

US-A-2011 160 725 offenbart eine Vorrichtung zur Steuerung eines Behandlungsvorgangs, wobei in einer ersten Behandlungsphase eine konstante Leistungsregelung verwendet wird. Bei Erfüllung eines Kriteriums wird von Leistungsregelung auf Spannungsregelung umgeschaltet.

Allerdings können bei vorzeitigem oder verspätetem Übergang von der ersten Behandlungsphase auf die zweite Behandlungsphase Probleme auftreten.

Der Erfindung liegt die Aufgabe zugrunde, eine Vorrichtung zu schaffen, mit dem sich eine Materialbehandlung wie beispielsweise eine Gewebebehandlung effektiv durchführen lassen.

Mit der Erfindung wird eine Vorrichtung gemäß Anspruch 1 oder einem oder mehreren der Vorrichtungsunteransprüche geschaffen.

Die nachstehend beschriebenen oder gezeigten Merkmale und Ausgestaltungen von Ausführungsbeispielen der erfindungsgemäßen Vorrichtung gelten jeweils auch umgekehrt für mögliche Ausgestaltungsformen der erfindungsgemäßen Vorrichtung, selbst wenn dies nicht ausdrücklich angegeben ist.

Die Erfindung betrifft mit einem Aspekt eine Vorrichtung zur Steuerung eines Behandlungsvorgangs, die ein Behandlungswerkzeug, insbesondere ein Elektrofusionsgerät wie etwa ein HF-Chirurgisches Instrument und eine Impedanzerfassungseinrichtung aufweist. Mittels einer Energiequelle wird elektrische Leistung in das durch das Behandlungswerkzeug zu behandelnde Material eingetragen. Eine Steuereinrichtung zur Steuerung der Energiequelle ist dazu ausgelegt, die Energiequelle derart zu steuern, dass in einer ersten Behandlungsphase eine Regelung der in das zu behandelnde Material eingespeisten Leistung erfolgt, und zwar bevorzugt mit einem rampenförmigen, z.B. linearen, exponentiellen, sigmoidalen, logarithmischen, Verlauf. Die Regelung der Leistung anstelle der Spannung stellt sicher, dass kein zu hoher Leistungseintrag in das Gewebe erfolgen kann, andererseits aber auch eine wirksame beherrschbare Einwirkung auf das Gewebe erfolgt und die Impedanz stabil verringert werden kann.

Die Steuereinrichtung der Vorrichtung ist dazu ausgelegt, die Energiequelle derart zu steuern, dass während oder nach der ersten Behandlungsphase bei Erfüllung eines integralen Kriteriums von Leistungsregelung auf Spannungsregelung bzw. Impedanzregelung umgeschaltet wird. Bei einem oder mehreren der beschriebenen Ausführungsbeispiele erfolgt eine Impedanzregelung über die Spannungsregelung.

Die Impedanzerfassungseinrichtung kann den Impedanzverlauf und/oder die aktuelle Impedanz des zu behandelnden Materials ermitteln und das Erreichen eines Impedanzminimums zuverlässig erkennen. Hierbei kann bei einem oder mehreren der beschriebenen Ausführungsbeispiele der Einsatz von digitalen oder analogen Filtern (z.B. Tiefpassfilter) vorgesehen sein, um schnelle Transienten aus dem Impedanzsignal auszufiltern.

Optional wird ein Zeitgeber in regelmäßigen oder unregelmäßigen Abständen, und / oder bei Erkennen eines Impedanzminimums gestartet, wobei der Zeitgeber auf ein bestimmtes Zeitintervall eingestellt ist. Der Zeitgeber kann bei Erkennen einer Impedanzverringerung oder eines ersten oder weiteren Impedanzminimums innerhalb des bestimmten Zeitintervalls wieder rückgesetzt werden und erneut zu laufen beginnen. Wenn kein neues Impedanzminimum innerhalb des bestimmten Zeitintervalls erkannt wird und das Zeitintervall abläuft, generiert der Zeitgeber ein Signal, das die Steuereinrichtung vorzugsweise zur Umschaltung der Leistungsregelung auf konstante Leistung oder optional auf einen Leistungsverlauf mit veränderter, z.B. stark verringerter Steigung oder abfallender Leistung veranlasst. Dieses Kriterium muss nicht zwingend während einer HF-Behandlung erreicht werden, sondern dient dem Schutz vor einem zu hohen Leistungseintrag, wenn die Gewebetemperatur nahe der Verdampfungstemperatur ist.

Bei Erfüllung des nachstehend noch näher beschriebenen, integralen Kriteriums erfolgt eine Umschaltung von Leistungsregelung auf Spannungsregelung, so dass nun die elektrische Spannung die geregelte Größe ist. Damit können unerwünscht große oder zu kleine Spannungen verhindert werden, die die Behandlung ungünstig beeinflussen könnten. Hierbei ist es nicht erforderlich, dass zuvor eine Leistungsbeschränkung eingegriffen hat.

Bei einem oder mehreren der beschriebenen Ausführungsbeispiele sind die Vorrichtung und das Verfahren dazu ausgelegt, den zeitlichen Spannungsanstieg und oder Leistungsanstieg zu limitieren.

Die Impedanzerfassungseinrichtung kann dazu ausgelegt sein, den Impedanzverlauf und/oder die aktuelle Impedanz des zu behandelnden Materials zu ermitteln und das Erreichen eines Impedanzminimums zu erkennen, wobei bei einem oder mehreren der beschriebenen Ausführungsbeispiele ein Zeitgeber, der auf ein bestimmtes Zeitintervall eingestellt sein kann, in regelmäßigen oder unregelmäßigen Abständen und / oder bei Erkennen eines Impedanzminimums gestartet werden kann. Der Zeitgeber kann bei Erkennen einer Impedanzverringerung oder eines ersten oder weiteren Impedanzminimums innerhalb des bestimmten Zeitintervalls wieder rückgesetzt werden und erneut zu laufen beginnen, wobei der Zeitgeber dann, wenn kein neues Impedanzminimum innerhalb des bestimmten Zeitintervalls erkannt wird und das Zeitintervall abläuft, optional ein Signal generiert, das die Steuereinrichtung zur Umschaltung der Leistungsregelung auf konstante Leistung oder einen Leistungsverlauf mit veränderter Steigung veranlasst.

Bei einem oder mehreren der beschriebenen Ausführungsbeispiele kann die Vorrichtung dazu ausgelegt sein, von der Spannungsregelung wieder in die Leistungsregelung überzugehen, wenn die Impedanz für eine vorgegebene Zeit wieder abfällt und/oder ein neues Impedanzminimum erkannt wird.

Bei einem oder mehreren der beschriebenen Ausführungsbeispiele ist das integrale Kriterium ein zeitliches Integral über die Impedanzerhöhung gegenüber einer kleinsten gemessenen Impedanz. Hierbei kann von Leistungsregelung auf Spannungsregelung umgeschaltet werden, wenn das zeitliche Integral einen Schwellenwert erreicht, so dass das integrale Kriterium auf sehr schnelle und auch auf langsame Impedanzumschläge oder Impedanzveränderungen reagieren kann.

Optional kann die Vorrichtung oder das Verfahren dazu ausgelegt sein, bei Umschaltung von Leistungsregelung auf Spannungsregelung die gerade angelegte Spannung zu erfassen und den für die Spannungsregelung zu verwendenden Spannungssollwert um einen bestimmten Wert oder Prozentsatz von beispielsweise 2 bis 70 %, vorzugsweise 5 bis 20 % zu verringern, so dass der für die Spannungsregelung zu verwendende Spannungssollwert dann z.B. bei 50 % bis 98 %, oder 80 % bis 95 %, der zum Umschaltzeitpunkt vorhandenen Spannung liegt.

Optional kann die Vorrichtung oder das Verfahren auch dazu ausgelegt sein, in einer anfänglichen Phase die initiale Gewebeimpedanz, vorzugsweise mit sehr kleiner Leistung, zu messen, so dass noch keine Impedanzänderung aufgrund einer Temperaturänderung geschieht.

Optional kann die Vorrichtung oder das Verfahren auch dazu ausgelegt sein zur Umschaltung auf Konstanthaltung der Leistung ein integrales Kriterium zu verwenden, wozu das zeitliche Integral über die Impedanzerhöhung gegenüber der kleinsten Impedanz, welche bislang gemessen wurde, ermittelt und umgeschaltet wird, sobald das zeitliche Integral ($Zs(n)$) einen Schwellenwert erreicht. Hierbei kann ein Quotienten aus dem zeitlichen Integral und der kleinsten Impedanz gebildet werden, um eine Normierung auf ein jeweiliges Impedanzlevel zu erreichen.

Optional kann die Vorrichtung oder das Verfahren auch dazu ausgelegt sein, nach einem Übergang in eine Haltephase eine Impedanz-Beschleunigung durchzuführen, bei der die Steigung der Impedanz ($Z$) erhöht wird, z.B. linear, wobei optional zur Regelung der Impedanz die HF-Spannung U als Stellgröße dienen kann.

Gemäß einem weiteren Aspekt, der nicht Teil der Erfindung ist, wird bei dem Verfahren zur Steuerung eines Behandlungsvorgangs mit einem Behandlungswerkzeug, insbesondere einem HF-Chirurgischen Instrument, eine Energiequelle derart gesteuert, dass in einer ersten Behandlungsphase eine Regelung der in das zu behandelnde Material eingespeisten Leistung mit einem ansteigenden, z.B. rampenförmigen, vorzugsweise linearen, Verlauf erfolgt, dann, optional bei Ansprechen eines Kriteriums, das erkennt, dass die Impedanz für eine vorbestimmte Zeit nicht mehr abgefallen oder nur schwach abgefallen ist, auf eine Leistungsregelung mit konstanter Leistung oder einem Leistungsverlauf mit veränderter Steigung umgeschaltet wird, und danach nach einem weiteren Zeitintervall von der Leistungsregelung auf eine Spannungsregelung umgeschaltet wird.

Hierbei kann die aktuelle Impedanz des zu behandelnden Materials und / oder der Impedanzverlauf ermittelt werden und das Erreichen eines Impedanzminimums erkannt werden: Ein auf ein bestimmtes Zeitintervall eingestellter Zeitgeber kann in regelmäßigen oder unregelmäßigen Abständen und / oder bei Erkennen eines Impedanzminimums gestartet werden. Der Zeitgeber kann bei Erkennen einer Impedanzverringerung oder eines ersten oder weiteren Impedanzminimums innerhalb des bestimmten Zeitintervalls wieder rückgesetzt werden und erneut zu laufen beginnen. Der Zeitgeber kann optional dann, wenn kein neues Impedanzminimum innerhalb des bestimmten Zeitintervalls erkannt wird und das Zeitintervall abläuft, ein Signal generieren, das eine Umschaltung der Leistungsregelung auf konstante Leistung oder einen Leistungsverlauf mit veränderter Steigung veranlasst. In einer Ausführungsvariante wird die Leistung bzw. der Leistungsanstieg als Funktion der Startimpedanz und der aktuellen Impedanz vorgegeben. Vorzugsweise wird hierzu das Verhältnis zwischen aktueller Impedanz und Startimpedanz verwendet.

Während der Leistungsregelung kann bei einem oder mehreren der beschriebenen Ausführungsbeispiele eine Limitierung des zeitlichen Spannungsanstiegs vorgenommen werden. Hierdurch wird eine Leistungslimitierung bzw.

Leistungsreduktion erzielt, wenn die Gewebeimpedanz schnell bzw. schlagartig ansteigt. Dies verhindert, bzw. eliminiert das Auftreten von Gewebeplatzern.

Die Vorrichtung kann zudem eingerichtet sein, um von der Spannungsregelung wieder in die Leistungsregelung überzugehen, wenn die Impedanz für eine vorgegebene Zeit wieder abfällt und/oder ein neues Impedanzminimum erkannt wird.

Bei Umschaltung von Leistungsregelung auf Spannungsregelung kann die gerade angelegte Spannung um einen bestimmten Wert oder Prozentsatz von beispielsweise 2 bis 70 %, oder 5 % bis 20 % verringert werden.

Bei einem oder mehreren der beschriebenen Ausführungsbeispiele wird die Spannung bei Umschaltung von Leistungsregelung auf Spannungsregelung so geändert, dass sich die Leistung um einen Prozentsatz von beispielsweise 1 bis 70% ändert.

Bei einem oder mehreren der beschriebenen Ausführungsbeispiele wird optional in einer anfänglichen Phase die initiale Gewebeimpedanz, vorzugsweise mit sehr kleiner Leistung, gemessen, wobei die initiale Gewebeimpedanz in der anfänglichen Phase beispielsweise mit einer Leistung gemessen wird, die beispielsweise nur 0,1 bis 3 Watt, oder 0,1 bis 10% der bei der nachfolgenden Leistungsregelung anfänglich verwendeten Leistung beträgt.

Der Leistungsanstieg kann bei einem oder mehreren der beschriebenen Ausführungsbeispiele eine Funktion des aktuellen Werts des zeitlichen Integrals sein.

Weiterhin kann der Leistungsanstieg bei einem oder mehreren der beschriebenen Ausführungsbeispiele in der ersten Behandlungsphase als Funktion, d.h. abhängig von, der Startimpedanz und/oder der aktuellen Impedanz gewählt werden.

Bei einem oder mehreren der beschriebenen Ausführungsbeispiele kann der Leistungsanstieg in der ersten Behandlungsphase z.B. in Abhängigkeit von dem Verhältnis von aktueller Impedanz zur Startimpedanz gewählt werden. Die Leistung bzw. der Leistungsanstieg kann bei einem oder mehreren der beschriebenen Ausführungsbeispiele beispielsweise als Funktion der Startimpedanz und der aktuellen Impedanz vorgegeben werden, wozu hierzu vorzugsweise das Verhältnis zwischen aktueller Impedanz und Startimpedanz verwendet wird.

Während der ersten Behandlungsphase, d.h. der Leistungsregelung kann bei einem oder mehreren der beschriebenen Ausführungsbeispiele eine Limitierung des zeitlichen Spannungsanstiegs vorgenommen werden.

Bei einem oder mehreren der beschriebenen Ausführungsbeispiele kann von der Spannungsregelung wieder in die Leistungsregelung übergegangen werden, wenn die Impedanz für eine vorgegebene Zeitdauer wieder abfällt und/oder ein neues Impedanzminimum erkannt wird.

Die Erfindung wird nachstehend anhand von Ausführungsbeispielen unter Bezugnahme auf die Zeichnungen näher beschrieben.

Figur 1 zeigt ein Ausführungsbeispiel einer erfindungsgemäßen Vorrichtung;

Figur 2 zeigt einen Verlauf der Gewebeimpedanz während einer Behandlung;

Figur 3 zeigt Einzelheiten einer Ausführungsform eines Verfahrens; und

Figur 4 zeigt einen schematischen Ablauf eines Behandlungsvorgangs unter Einsatz eines Ausführungsbeispiels der erfindungsgemäßen Vorrichtung.

Zunächst wird ein integrales Kriterium zur Erkennung eines Impedanzminimums beschrieben, das bei einem Ausführungsbeispiel der erfindungsgemäßen Vorrichtung eingesetzt werden kann oder wird.

Hierzu wird im Folgenden zunächst das Verhalten von biologischem Gewebe während einer Behandlung mit hochfrequentem Wechselstrom von beispielsweise 300 kHz bis 1 MHz beschrieben, wie es in der HF-Behandlung üblich ist (HF steht für Hochfrequenz). Das zu behandelnde Gewebe kann auch nicht-biologischer Art sein. Hierbei werden die Temperaturen, das Impedanzverhalten und Gewebemodifikationen betrachtet, um Eigenheiten eines thermischen Behandlungsverfahrens mittels Hochfrequenz bzw. eines Fusionsprozesses zu veranschaulichen.

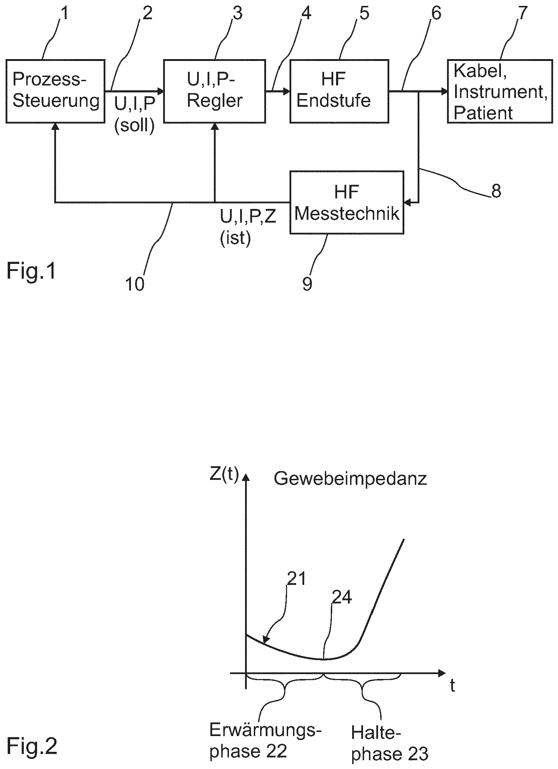

Allgemein ergibt sich während der Erwärmung von biologischem oder auch nichtbiologischem Gewebe, beispielsweise durch Einsatz von elektrischem Strom, ein charakteristisches Verhalten der Impedanz, wie es exemplarisch in Figur 2 gezeigt ist. Auf der Ordinate des Schaubilds gemäß Figur 2 ist die Impedanz Z aufgetragen, während auf der Abszisse die Zeit t aufgetragen ist. Die Impedanzkurve ist mit dem Bezugszeichen 21 versehen. In Figur 2 ist die Erwärmungsphase mit dem Bezugszeichen 22 bezeichnet, während die Haltephase mit der Bezugsziffer 23 versehen ist.

Zunächst fällt die Impedanz in aller Regel ab, was dadurch begründet sein kann, dass es sich bei biologischem Gewebe um einen Ionenleiter handelt, welcher einen negativen Temperaturkoeffizienten aufweist. Die Impedanz verringert sich in einem solchen Fall bis zu jenem Punkt, an dem ein Verdampfen der Gewebeflüssigkeiten einsetzt.

Dies ist in der Regel der niedrigste Punkt 24 der in Figur 2 gezeigten, auch als Badewannenkurve bezeichneten Impedanzkurve 21, und wird beispielsweise bei einer Gewebetemperatur von ca. 90° C erreicht. Bei weiterem Erhitzen des Gewebes steigt die Impedanz Z in der Regel nun wieder fortlaufend an. Dies resultiert daraus, dass der durch die Verdampfung gebildete Wasserdampf eine schlechte Leitfähigkeit besitzt und das leitfähige Wasser aus dem Gewebe ausgetrieben wird, so dass eine Austrocknungsphase vorliegt. Diese beiden Phasen können beispielsweise als Erwärmungsphase (Heating-Phase) 22 und Haltephase (Keeping-Phase) 23 bezeichnet werden. In der Erwärmungsphase 22 findet folglich die Gewebeerwärmung bis zum Punkt der Wasserverdampfung statt, woraufhin in der nachfolgenden Haltephase 23 die Temperatur gehalten bzw. nur noch geringfügig gesteigert wird.

Allgemein stellt der Zeitpunkt 24 des Übergangs von der Erwärmungsphase 22 in die Haltephase 23 einen kritischen Punkt bei der Steuerung und Regelung eines HF-Behandlungsprozesses dar. Zu diesem Zeitpunkt 24 wird nämlich die Siedetemperatur des Gewebewassers erreicht, so dass dieses sehr schnell von der flüssigen Phase in die Dampfphase übergeht. Es wurde erkannt, dass es aufgrund von schlagartig entweichendem Dampf zum Aufplatzen des Gewebes kommen kann, wenn die HF-Leistung (Hochfrequenzleistung) zu diesem Zeitpunkt nicht rechtzeitig reduziert wird. Beim Aufplatzen ergibt sich als elektrisches Charakteristikum ein exponentieller Impedanzanstieg. Ein zu rascher Übergang von der Erwärmungsphase 22 auf die Haltephase 23 kann zudem die Gefahr von erhöhten thermischen Beschädigungen ("Thermal Spread") mit sich bringen, da rasch entweichender Wasserdampf umliegendes Gewebe thermisch schädigen kann.

Wie aus Figur 2 ersichtlich ist, sinkt die Impedanz Z in der Erwärmungsphase 22 (Phase 1) zunächst ab, erreicht ihr Minimum und steigt in der Haltephase 23 (Phase 2) oftmals schlagartig wieder an. Da in der Erwärmungsphase 22 (Phase 1) und in der Haltephase 23 (Phase 2) unterschiedliche Methoden für die Steuerung und Regelung verwendet werden können, wird bei den hier beschriebenen Ausführungsbeispielen angestrebt, den Übergangspunkt 24 von Phase 1 in Phase 2 präzise zu erkennen, um zwischen den beiden Steuerungsprogrammen bzw. Methoden wechseln zu können oder eine entsprechende Steuerungsverfahrensumsteuerung durchzuführen. Allgemein hat sich gezeigt, dass die Erkennung des Übergangspunkts 24 problematisch ist, da sich abhängig von Gewebetyp, Gewebemenge, Elektrodenfeuchtigkeit usw. stark unterschiedliche Verhaltensweisen ergeben können. Eine fehlerhafte Erkennung führt zu unerwünschten Effekten bei dem Prozessfortschritt, wobei zwischen einer vorzeitigen und einer verzögerten Prozessumschaltung unterschieden werden kann.

Bei einer vorzeitigen Umschaltung wird beispielsweise bei vermeintlicher Erkennung eines Impedanzminimums in die zweite Steuerungsphase (Phase 2) umgeschaltet, bei der eine Regelung des Impedanzverlaufs durchgeführt werden kann. Bei vorzeitiger Umschaltung erwartet die Regelung ein Ansteigen der Impedanz bei Erhöhung des Leistungseintrags, z.B. durch Erhöhung des Stroms und / oder der Spannung und / oder der Behandlungszeitintervalle. Da bei fehlerhafter Impedanzminimumeinstufung die Impedanz trotz höherem Leistungseintrag jedoch weiter abfällt, wird die Abweichung zwischen der eigentlich gewünschten Soll-Trajektorie des Impedanzverlaufs und der tatsächlich vorliegenden Ist-Trajektorie des Impedanzverlaufs zunehmend größer. Dies führt zu einem überhöhten Leistungseintrag, wodurch Gewebeschäden hervorgerufen werden können.

Andererseits wird bei einer verzögerten Umschaltung trotz bereits erfolgtem Durchlaufen des Impedanzminimums und wieder ansteigender Impedanz (in manchen Fällen sogar schlagartig) doch noch keine Umschaltung in die nächste Steuerungsphase (Phase 2) vorgenommen, da beispielsweise noch nicht alle Bedingungen für das Umschalten erfüllt sind. In einer solchen Situation ergibt sich die Gefahr, dass das Gewebe wegen der nicht reduzierten Leistung aufplatzt, da das Wasser schlagartig verdampft und / oder ausgetrieben wird. Als Folge können sich Gewebeschäden in Form von aufgeplatztem Gewebe ebenso wie starkes Thermal Spread und ebenso denaturiertes Gewebe ergeben, das dann aufgrund der nun fehlenden Flüssigkeit oder Feuchtigkeit nicht mehr versiegelt werden kann.

Als Nachteile bei einer solchen vorzeitigen oder verzögerten Umschaltung zwischen den Steuerungsphasen können sich somit Gewebeschäden in Form von aufgeplatztem Gewebe, karbonisiertem Gewebe, denaturiertem / ausgetrocknetem Gewebe, Gewebeanhaftung an Elektroden, und starker Thermal Spread ergeben.

Eine unerwünschte, fehlerhafte Umschaltung zwischen den Steuerungsphasen kann durch verschiedenartige Probleme oder Fehler hervorgerufen werden. Beispielsweise kann in Folge eines Gewebeplatzers ein sprunghafter Anstieg der Impedanz auftreten, wonach die Impedanz ähnlich wie bei der Phase 1 wieder abklingt. Dieser Impedanzsprung begründet aber die Gefahr einer fehlerhaften vorzeitigen Erkennung und Einstufung als Minimum mit Umschaltung der Steuerung.

Weiterhin kann der Fall auftreten, dass das Gewebe mehrere Minima durchläuft, was beispielsweise bei Gewebetypen der Fall sein kann, die aus zwei oder mehreren strukturell unterschiedlichen Schichten bestehen. So zeigen beispielsweise Gewebe des Ösophagus oder Teile des Colons in der Phase 1 häufig zwei oder mehrere Impedanz-Zwischenminima. In einem solchen Fall durchläuft die Impedanz im Allgemeinen das erste Minimum, steigt daraufhin wieder über mehrere Sekunden an und fällt dann jedoch nochmals weiter unter das erste Minimum ab. Die Verhältnisse der Minima und ihre zeitlichen Abstände können hierbei gewebeabhängig stark variieren. Auch in einem solchen Fall besteht die Gefahr einer vorzeitigen Umschaltung in die Phase 2, obwohl die Phase 1 de facto noch nicht abgeschlossen ist.

Als weiterer Störfall kann ein plötzlicher Impedanzumschlag auftreten, bei dem insbesondere bei der Behandlung einer geringen Gewebemenge der Impedanzverlauf im Sinne der Impedanzkurve 21 gemäß Figur 2 oftmals schlagartig ansteigt. Dies zeichnet sich in einem plötzlichen Impedanzumschlag ab. Steuerungstechnisch könnte ein solches Ereignis aber als Gewebeplatzer im vorstehenden Sinn erkannt werden und die Umschaltung hierdurch verzögert werden.

In Figur 1 ist ein Ausführungsbeispiel einer erfindungsgemäßen Vorrichtung in Form eines Blockschaltbilds gezeigt, das eine einen solche Fehler vermeidende Prozesssteuerung und Prozessregelung veranschaulicht. Fig. 1 zeigt somit ein Blockschaltbild der Prozesssteuerung / Regelung.

Das Ausführungsbeispiel gemäß Figur 1 weist eine Prozesssteuerung 1, einen Regler 3, eine Hochfrequenz-Endstufe 5 und ein schematisch angedeutetes Instrument 7 sowie eine Messeinrichtung 9 auf. Die Prozesssteuerung 1 gibt über eine Signalverbindung 2, beispielsweise in Form einer Leitung oder einer drahtlosen Kommunikation, Eingangsgrößen an den Regler 3 ab. Bei einem oder mehreren der beschriebenen Ausführungsbeispiele können die Prozesssteuerung 1 und der U,I,P Regler 3 in einem Mikroprozessor untergebracht sein. Die Eingangsgrößen können beispielsweise ein oder mehrere Sollwerte für die Spannung U, den Strom I und / oder die Leistung P sein. Der Regler 3 kann beispielsweise als Spannungsregler und / oder Stromregler und / oder Leistungsregler agieren und gibt sein oder seine Ausgangssignale über eine Signalverbindung 4, beispielsweise in Form einer Leitung oder drahtlosen Kommunikation, an die Hochfrequenz-Endstufe 5 ab, die die Treibersignale für das Instrument 7 über eine Signalverbindung 6, beispielsweise in Form eines Kabels oder einer drahtlosen Kommunikationsstrecke, ausgibt. Die ausgangsseitig von der HF-Endstufe 5 ausgegebenen, an das Instrument 7 anzulegenden Signale und / oder von dem Instrument 7 erhaltene Signale werden über eine Signalverbindung 8, beispielsweise in Form einer Leitung oder einer drahtlosen Kommunikationsverbindung, an die Messvorrichtung 9 (HF-Messung) angelegt, die die an das Instrument 7 angelegten und / oder von diesem abgegebenen Signale misst und ausgangsseitig die aktuell gemessenen Ist-Werte der Spannung U und / oder des Stroms I und / oder der Leistung P und / oder der Impedanz Z über die Signalverbindung 10, beispielsweise in Form einer Leitung oder einer drahtlosen Kommunikationsstrecke, an eine mit einem Eingang des Reglers 3 verbundene Signalverbindung 11 beispielsweise in Form einer Leitung oder einer drahtlosen Kommunikation, ausgibt und über diese an einen Eingang des Reglers 3 sowie an einen Eingang 12 der Prozesssteuerung 1 anlegt. Damit erhalten sowohl die Prozesssteuerung 1 als auch der Regler 3 Informationen über die aktuellen Werte der an das Instrument 7 angelegten bzw. dort gemessenen Ist-Werte des Stroms und / oder der Spannung und / oder der Leistung und / oder der Impedanz.

Bei dem gezeigten Ausführungsbeispiel werden die aktuellen Spannungen, Ströme, die Leistung sowie die Gewebeimpedanz von der HF-Messvorrichtung 9, die sich zwischen der HF-Endstufe 5 und dem Instrument 7 bzw. dem Patienten befindet, erfasst und an die Prozesssteuerung 1 weitergegeben. Die Prozesssteuerung 1 agiert in unterschiedlichen Prozessphasen (Phase 1, also 22, und Phase 2, also 23) als Leistungssteuerung oder als Impedanzregler. Die Prozesssteuerung 1 liefert die Vorgabewerte für Spannung, Strom und Leistung für den U, I, P - Regler 3, der die HF-Endstufe 5 derart regelt, dass keiner der Vorgabewerte für die Spannung, den Strom und / oder die Leistung überschritten wird. Die Prozesssteuerung 1 und der Regler 3 können daher als kaskadierter Regelkreis agieren, in welchem die Prozesssteuerung 1 den Regler des äußeren Regelkreises bildet, während der U, I, P - Regler 3 den Regler des inneren Regelkreises darstellt.

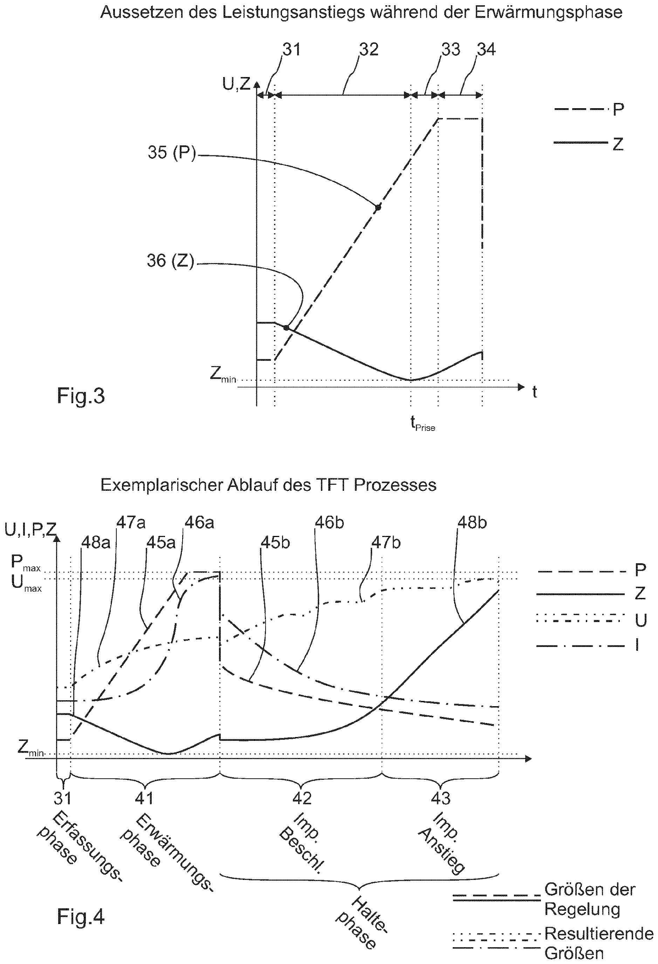

Im Folgenden wird die Arbeitsweise von Ausführungsbeispielen der erfindungsgemäßen Vorrichtung anhand Fig. 4 exemplarisch beschrieben. Fig. 4 zeigt den typischen Verlauf eines Versiegelungsprozesses für die Leistung P, Kurve 45a, 45b; den Strom I, Kurve 46a, 46b; die Spannung U, Kurve 47a, 47b; und die Gewebeimpedanz Z, Kurve 48a, 48b. Diese Größen sind miteinander verknüpft, so dass sie nicht unabhängig voneinander variiert werden können. Die für die Regelung verwendeten Größen sind daher in Fig. 4 jeweils mit Linien 45a, 48b eingezeichnet, wohingegen die daraus resultierenden Größen mit Linien 45b, 46a, 46b, 47a, 47b, 48a dargestellt sind. In der Heating-Phase 41 wird die Leistung P geregelt (Kurve 45a) und die Impedanz Z beobachtet (Kurve 48a), wohingegen in der Keeping-Phase 42, 43 die Impedanz Z (Kurve 48b) unter Verwendung der Spannung U (Kurve 47b) als Stellgröße geregelt wird.

Im Folgenden wird eine Ausführungsform eines Verfahrens näher erläutert, die mit dem in Figur 1 dargestellten Ausführungsbeispiel der erfindungsgemäßen Vorrichtung durchführbar ist.

In einem optionalen ersten Schritt wird eine kurzzeitige Erfassung durchgeführt, die auch als Erfassungs- oder Sensing-Phase bezeichnet werden kann. Während dieser ersten Phase findet eine Messung der initialen Gewebeimpedanz statt. Die Messung kann sehr kurzfristig durchgeführt werden und beispielsweise nur wenige Millisekunden von beispielsweise 1 bis 500 Millisekunden ausgeführt werden. Die Leistung P während dieses ersten Schritts wird hierbei so klein gewählt, dass kein thermischer Gewebeeffekt hervorgerufen wird. Die Leistung kann beispielsweise ca. 0,1 bis 5 Watt betragen. Allgemein ist die Leistung $P(n) = P_m$ in dieser Phase ($n \varepsilon N$) entsprechend klein gewählt. Der in dieser Phase gemessene Impedanzwert kann für den weiteren Prozessverlauf verwendet werden. Diese Phase wird für eine vorgegebene Messdauer von beispielsweise 1 bis 500 ms, also für die vorgegebene Messdauer durchgeführt. Damit ist diese erste Phase, das heißt der erste Schritt beendet.

In einem zweiten Schritt wird dann eine Erwärmungsphase 22 (heating phase) als zweite Phase durchgeführt, wobei das Verfahren allerdings auch direkt mit dieser zweiten Phase, das heißt der Erwärmungsphase starten kann, ohne die erste Phase auszuführen.

Ausgehend von der beim ersten Schritt angewandten, oder zu Beginn der Erwärmungsphase angewandten Messleistung $P_m$ wird die im Folgenden auch abgekürzt als HF-Leistung bezeichnete Hochfrequenzleistung, die an das Instrument angelegt wird, bis zum Erreichen eines Impedanzminimums erhöht. Diese Erhöhung kann linear erfolgen, aber in anderen Fällen auch nichtlinear. Damit ergibt sich der folgende formelmäßige Zusammenhang:

$$P(n) = P_m + \alpha_{PR} T_s * n$$

In der vorgenannten Gleichung steht der Faktor $\alpha_{PR}$ für die Leistungssteigerung, während $T_s$ die Abtastzeit angibt. In dieser Phase wird besondere Aufmerksamkeit auf den vorstehend bereits angesprochenen, kritischen Punkt 24 des Phasenübergangs gelegt, da in dieser Prozessphase, das heißt der Erwärmungsphase, erhöhte Gefahr von Gewebeschäden besteht. In dieser Phase, das heißt dem zweiten Schritt, wird eine ansteigende HF-Leistung verwendet, da zum Startzeitpunkt noch nicht bekannt ist, wie viel Gewebe mit dem Instrument 7 gefasst wurde. Durch diese ansteigende HF-Leistung wird im Gegensatz zu einer konstanten Leistung verhindert, dass die Erwärmungsphase sehr lange andauert, wenn die Leistungseinstellung zu gering ist. Dies würde die Gefahr von Gewebeanhaftungen mit sich bringen. Zugleich wird durch die ansteigende HF-Leistung auch verhindert, dass die Erwärmungsphase zu schnell, beispielsweise in Sekundenbruchteilen, durchlaufen wird und das Gewebe aufplatzt, wenn die Hochfrequenzleistung (bei konstanter Leistung) zu hoch gewählt sein sollte.

Durch diese bei dem Ausführungsbeispiel vorgesehene Vorgehensweise wird die richtige Leistungseinstellung gewissermaßen automatisch gesucht und gefunden.

Um den vorstehend bereits beschriebenen schlagartigen Impedanzumschlag zu verhindern, ist bei einem oder mehreren Ausführungsbeispielen der Erfindung vorgesehen, dass die Erhöhung der Hochfrequenzleistung nicht mehr fortgeführt wird, wenn die bislang abfallende Gewebeimpedanz wieder ansteigt bzw. für eine vorgegebene Zeit von beispielsweise $t_{P\ rise}$ keinen neuen Minimalwert mehr angenommen hat. Zur Erfassung der zeitlichen Dauer seit dem letzten erfassten Impedanzminimum ist eine Variable $t_{lastZmin}$ vorgesehen. Demzufolge ergibt sich als Formel zur Steuerung der HF-Leistung die folgende Gleichung:

$$P(n) = P(n-1) + \frac{a}{T_s} \begin{cases} a = 0, & t_{last\ Zmin} > t_{P\ rise} \\ a = a_{PR}, & sonst \end{cases}$$

Die HF-Leistung wird also konstant gehalten und steigt nicht länger an, wenn für ein definiertes Zeitintervall $t_{P\ rise}$ keine neue kleinere Minimalimpedanz mehr gemessen wurde, das heißt die Impedanz gleich geblieben oder sogar wieder angewachsen ist. In diesem Fall kann daher davon ausgegangen werden, dass sich der Prozess nahe bei dem "kritischen Phasenübergang" befindet.

Figur 3 zeigt ein exemplarisches Beispiel für die Leistungssteuerung während der Erwärmungsphase (Phase 2, oder 22 in Fig. 2. Wie aus Figur 3 mit dem die Leistung anzeigenden Kurvenzug 31 ersichtlich ist, wird die zunächst im Wesentlichen linear ansteigende Leistungserhöhung auf einen konstanten Wert umgestellt, der bei dem dargestellten Ausführungsbeispiel dem bislang erreichten Leistungswert entspricht, jedoch auch hiervon abweichend niedriger oder höher sein kann.

Der Zeitpunkt des Umschaltens auf die Konstanthaltung der in das Instrument 7 eingespeisten Leistung P, siehe Kurvenzug 31, liegt um das vordefinierte, durch das Bezugszeichen 33 veranschaulichte Zeitintervall $t_{Prise}$ nach dem Zeitpunkt der letzten Detektion eines Minimums, nach dem sich die durch den Kurvenzug 32 veranschaulichte Impedanz Z nicht weiter verringert, sondern im Gegenteil bei dem dargestellten Ausführungsbeispiel sogar wieder anwächst.

Bei dem Ausführungsbeispiel gemäß Figur 3 wird also der Leistungsanstieg, der während der Erwärmungsphase vorgesehen ist, dann ausgesetzt und die erreichte Leistung konstant beibehalten.

In Figur 3 sind mit den Bezugszeichen 31 bis 34 die erste, zweite, dritte bzw. vierte Phase der Leistungssteuerung bezeichnet. Die Kurve 35 bezeichnet den gesteuerten Verlauf der Leistung, während die Kurve 36 den Impedanzverlauf Z wiedergibt. Auf der Ordinate sind die Leistung P sowie die Impedanz Z aufgetragen, während auf der Abszisse die Zeit dargestellt ist. In der optionalen Phase 31 wird unter kurzzeitiger Konstanthaltung der Leistung die anfängliche Impedanz Z gemessen, wonach dann, nach Ablauf der vorgegebenen Zeitdauer, oder bei einem anderen Ausführungsbeispiel auch sofort bei Beginn der Behandlung, ein linearer Leistungsanstieg in der mit dem Bezugszeichen 32 bezeichneten Phase 2 vorgesehen ist. Der Übergang zu der mit dem Bezugszeichen 33 bezeichneten, dritten Phase mit noch weiter ansteigender Leistung P ist durch die Erfassung des Impedanzminimums $Z_{min}$ charakterisiert, wonach noch in der dritten Phase 33 abgewartet und überprüft wird, ob tatsächlich kein neues, tieferes Minimum erreicht wird. Nach Ablauf der Phase 33 wird dann in der vierten Phase 34 auf Konstanthaltung der Leistung P umgeschaltet.

Zur Beendigung der Phase 32, 33 dient ein integrales Kriterium, das auf sehr schnelle und auch auf langsame Impedanzumschläge oder Impedanzveränderungen reagieren kann.

Der Wert

$$Zs(n) = \sum_{n_{min}}^{n} ( Z(n) - Z_{min}) \cdot T_s \begin{cases} Z_{min} = Z(n) \\ n_{min} = n \end{cases} , Z(n) < Z_{min}$$

ist das zeitliche Integral über die Impedanzerhöhung gegenüber der kleinsten Impedanz $Z_{min}$, welche in einem Prozess zum Zeitpunkt n * Ts gemessen wurde. Die Erwärmungsphase wird beendet, sobald Zs(n) einen Schwellenwert erreicht. Zs(n) ergibt sich zu 0, wenn in einem Prozess eine neue Minimalimpedanz erreicht wird. Damit kann Zs(n) in diesem Fall nicht den vorgegebenen Schwellenwert erreichen.

In obiger Gleichung ist noch nicht berücksichtigt, dass ein Versiegelungsvorgang (Sealing-Prozess) abhängig vom Gewebetyp, dessen Zustand, Größe und Instrument etc. auf unterschiedlich hohen Impedanzlevels ablaufen kann. Um dies zu berücksichtigen lässt sich obige Gleichung umschreiben in

$$Zs(n) = \frac{\sum_{n_{min}}^{n} ( Z(n) - Z_{min}) \cdot T_s}{Z_{min}} \begin{cases} Z_{min} = Z(n) \\ n_{min} = n \end{cases} , Z(n) < Z_{min}$$

Nun ergibt sich Zs(n) aus dem Quotienten des besagten Integrals und $Z_{min}$. Hiermit wird eine Normierung auf das jeweilige Impedanzlevel, auf dem der Prozess abläuft, erreicht.

Nachdem der Prozess von der Erwärmungsphase (zweite Phase) oder Phase 41 in Fig. 4 in die Haltephase (Keeping-Phase) 42, 43 übergegangen ist, erfolgt zunächst in Phase 42 eine Absenkung der HF-Leistung um einen bestimmten Faktor (vgl. Fig. 4) von z. B. 30 % bis 70 %. Dies dient dazu, den gerade stattfindenden Impedanzumschlag abzufangen. Die sprunghafte Absenkung der Leistung bringt meist auch eine kleine Absenkung der Impedanz Z mit sich.

In dieser Phase 42, 43 des Prozesses erfolgt eine Regelung auf eine Impedanztrajektorie, welche sich gemäß Fig. 4 in zwei Abschnitt unterteilt:

- Impedanz-Beschleunigung in Phase 42
- Impedanz-Anstieg (optional mit konstanter Steigung) in Phase 43.

In der Phase 42 der "Impedanz-Beschleunigung" wird die Steigung der Impedanz Z linear erhöht. Diese Maßnahme dient der Minimierung von Thermal Spread, welches entstehen kann, wenn an dieser Stelle zu viel Wasser zu schnell verdampft wird. Die Dauer der Impedanz-Beschleunigung ist zeitlich vorgegeben. Die Phase 42 wird beendet, wenn eine vorgegebene Impedanz erreicht ist.

Zur Regelung der Impedanz dient die HF-Spannung U als Stellgröße. Zur Berechnung der HF-Ausgangsspannung

$$U_{HF}(n) = U_{HF}(n-1) + \frac{\Delta U_{HF}}{T_s} \begin{cases} \Delta U_{HF} = \Delta U_{HF\,pos}, & Z(n) < Z_{soll}(n) \\ \Delta U_{HF} = \Delta U_{HF\,neg}, & Z(n) > Z_{soll}(n) \end{cases}$$

wird unterschieden, ob sich die aktuelle Impedanz Z(n) über oder unterhalb der Sollimpedanz Zsoll befindet. Abhängig davon wird die Ausgangsspannung UHF um einen bestimmten Wert $\frac{\Delta U_{HF\,pos}}{T_s}$ oder $\frac{\Delta U_{HF\,neg}}{T_s}$ verändert.

Bei dem Ausführungsbeispiel wird somit mit einer Leistungsrampe gearbeitet, bei der die Leistung rampenförmig, also mit konstanter Steigung, erhöht wird, wobei die Leistungsrampe ausgesetzt wird, wenn lange kein neues Minimum erkannt wurde, wonach auf konstante Leistung umgeschaltet wird.

Die Leistung wird also in der Anfangsphase linear erhöht, bis das Gewebe aufgeheizt ist, wobei der Aufheizvorgang als abgeschlossen angesehen wird, wenn die Gewebeimpedanz nach ihrem Minimum wieder ansteigt.

Die Steilheit der Leistungskurve ist so gewählt, dass sich keine Gewebeaufplatzungen oder thermische Schädigungen bzw. eine schlagartige Austrocknung des Gewebes einstellen.

Bei dem Ausführungsbeispiel ist vorgesehen, die Hochfrequenzleistung P nur so lange zu erhöhen, wie sich die Gewebeimpedanz im Abwärtstrend befindet.

Hierzu wird ein Zeitgeber (timer) eingesetzt, der die Zeitdauer erfasst, wie lange das letzte Impedanzminimum zurückliegt. Der Zeitgeber wird jeweils bei Erfassung eines Impedanzminimums wieder auf Null gesetzt und beginnt dann erneut zu zählen. Der Zeitgeber ist auf ein bestimmtes Zeitintervall eingestellt und gibt bei seinem Ablauf ohne zwischenzeitliche Rücksetzung, das heißt also ohne zwischenzeitliche Erfassung eines neuen Impedanzminimums, ein Ausgangssignal ab, bei dessen Auftreten die Steuereinrichtung den rampenförmigen Leistungsanstieg beendet.

Der lineare Leistungsanstieg kann bei Erreichen und/oder Überschreiten eines Grenzwerts beendet werden. Der Grenzwert kann ein zeitlicher Grenzwert sein, das heißt dem durch den Zeitgeber vorgegebenen Zeitintervall entsprechen, kann aber auch ein Leistungsgrenzwert sein, bei dessen Erreichen der Leistungsanstieg beendet oder gegebenenfalls nur mit deutlich verringerter Steigung, also flacher weiter ausgeführt wird.

Bei einem weiteren Ausführungsbeispiel kann vorgesehen sein, die Zeitdauer zwischen dem Auftreten von Minima der Impedanz während der weiter linear anwachsenden Leistung zu erfassen. Wenn ermittelt wird, dass die Zeitdauer zwischen dem Auftreten von Impedanzminima größer wird, jedoch noch nicht die durch den Zeitgeber vorgegebene Zeitdauer erreicht, kann die erfindungsgemäße Vorrichtung bei einem oder mehreren Ausführungsbeispielen auch die Steigung der Leistungskurve verringern. Damit wird eine sanftere Annäherung an den Umschaltpunkt erreicht, bei dem dann, wenn der Zeitgeber tatsächlich abgelaufen ist, das heißt während des vorbestimmten Zeitintervalls kein neues Impedanzminimum detektiert worden ist, dann endgültig auf konstante Leistung umgeschaltet wird.

Wie aus Figur 4 ersichtlich ist, wird zum Ende der Erwärmungsphase 41 die Regelung der Leistung P (zunächst rampenförmig, dann konstant) beendet, wonach die Leistung P in den nachfolgenden Phasen 42 mit Impedanzbeschleunigung und 43 mit Impedanzanstieg nicht länger die geregelte Größe ist. Stattdessen wird zu diesem Zeitpunkt nun auf Spannungsregelung umgeschaltet, bei der die Spannung die Regelgröße ist und gezielt vom Mikroprozessor mit dem stufenförmig anwachsenden Verlauf erhöht wird. Jedoch ist unmittelbar beim Umschalten von Leistungsregelung mit der Leistung P als Regelgröße auf die nachfolgende Regelung der Spannung mit der Spannung U als Regelgröße gezielt eine Absenkung der Spannung U vorgesehen, wie dies beim Übergang zwischen den Kurvenästen 47a, 47b (Spannung U) ersichtlich ist.

In der Phase 41 ist somit lediglich die Leistung P die Regelgröße, wie dies durch den Kurvenzug 45a veranschaulicht ist, während die Impedanz Z, die Spannung U und der Strom I sich als Folge der lokalen Bedingungen und der gezielten Leistungsregelung einstellen, siehe Kurven 46a (I), 47a (U) und 48a (Z).

Während der Phasen 42, 43 ist lediglich die Spannung gemäß Kurve 47b gezielt geregelt, während sich die Kurven 45b (Leistung P), 46b (Strom I) und 48b (Impedanz Z) als Folge hiervon einstellen.

## Patentansprüche

1. Vorrichtung zur Steuerung eines Behandlungsvorgangs, mit einem Behandlungswerkzeug, insbesondere einem HF-Chirurgischen Instrument, einer Impedanzerfassungseinrichtung, einer Energiequelle zum Einbringen elektrischer Leistung in ein durch das Behandlungswerkzeug zu behandelndes Material, einem Zeitgeber und einer Steuereinrichtung zur Steuerung der Energiequelle,
   wobei die Steuereinrichtung dazu ausgelegt ist, die Energiequelle derart zu steuern, dass in einer ersten Behandlungsphase eine Regelung der in das zu behandelnde Material eingespeisten Leistung mit einem ansteigenden, z. B. rampenförmigen Verlauf erfolgt,
   und wobei bei Erfüllung eines integralen Kriteriums von Leistungsregelung auf Spannungsregelung oder Impedanzregelung umgeschaltet wird.

2. Vorrichtung nach Anspruch 1, die dazu ausgelegt ist, den zeitlichen Spannungsanstieg zu limitieren.

3. Vorrichtung nach Anspruch 1 oder 2, wobei die Impedanzerfassungseinrichtung dazu ausgelegt ist, den Impedanzverlauf und/oder die aktuelle Impedanz des zu behandelnden Materials zu ermitteln und das Erreichen eines Impedanzminimums zu erkennen,
   wobei der Zeitgeber in regelmäßigen oder unregelmäßigen Abständen gestartet wird und / oder bei Erkennen eines Impedanzminimums gestartet wird und auf ein bestimmtes Zeitintervall eingestellt ist,

wobei der Zeitgeber bei Erkennen einer Impedanzverringerung oder eines ersten oder weiteren Impedanzminimums innerhalb des bestimmten Zeitintervalls wieder rückgesetzt wird und erneut zu laufen beginnt, und wobei der Zeitgeber dann, wenn kein neues Impedanzminimum innerhalb des bestimmten Zeitintervalls erkannt wird und das Zeitintervall abläuft, ein Signal generiert, das die Steuereinrichtung zur Umschaltung der Leistungsregelung auf konstante Leistung oder einen Leistungsverlauf mit veränderter Steigung veranlasst.

4. Vorrichtung nach einem der vorhergehenden Ansprüche, die dazu ausgelegt ist, von der Spannungsregelung wieder in die Leistungsregelung überzugehen, wenn die Impedanz für eine vorgegebene Zeit wieder abfällt und/oder ein neues Impedanzminimum erkannt wird.

5. Vorrichtung nach einem der vorhergehenden Ansprüche, bei der das integrale Kriterium ein zeitliches Integral über die Impedanzerhöhung gegenüber einer kleinsten gemessenen Impedanz ist, und von Leistungsregelung auf Spannungsregelung umgeschaltet wird, wenn das zeitliche Integral einen Schwellenwert erreicht, so dass das integrale Kriterium auf sehr schnelle und auch auf langsame Impedanzumschläge oder Impedanzveränderungen reagieren kann.

6. Vorrichtung nach einem der vorhergehenden Ansprüche, die dazu ausgelegt ist, bei Umschaltung von Leistungsregelung auf Spannungsregelung die gerade angelegte Spannung um einen bestimmten Wert oder Prozentsatz von beispielsweise 2 bis 70 %, oder 5 % bis 20 % zu verringern.

7. Vorrichtung nach einem der vorhergehenden Ansprüche, die dazu ausgelegt ist, in einer anfänglichen Phase die initiale Gewebeimpedanz, vorzugsweise mit sehr kleiner Leistung, zu messen.

8. Vorrichtung nach einem der vorhergehenden Ansprüche, die dazu ausgelegt ist, zur Umschaltung auf Konstanthaltung der Leistung ein integrales Kriterium zu verwenden; wobei das zeitliche Integral über die Impedanzerhöhung gegenüber der kleinsten Impedanz, welche bislang gemessen wurde, ermittelt und umgeschaltet wird, sobald das zeitliche Integral (Zs(n)) einen Schwellenwert erreicht.

9. Vorrichtung nach Anspruch 8, die dazu ausgelegt ist, einen Quotienten aus dem zeitlichen Integral und der kleinsten Impedanz zu bilden, um eine Normierung auf ein jeweiliges Impedanzlevel zu erreichen.

10. Vorrichtung nach Anspruch 8, die dazu ausgelegt ist, einen Quotienten aus dem zeitlichen Integral und der Startimpedanz zu bilden, um eine Normierung auf ein jeweiliges Impedanzlevel zu erreichen.

11. Vorrichtung nach einem der vorhergehenden Ansprüche, mit einem digitalen und/oder analogen Filter, wobei die aktuelle Gewebeimpedanz durch Filterung mittels des Filters ermittelt wird.

12. Vorrichtung nach einem der vorhergehenden Ansprüche, die dazu ausgelegt ist, nach einem Übergang in eine Haltephase eine Impedanz-Beschleunigung durchzuführen, bei der die Steigung der Impedanz (Z) erhöht wird, z. B. linear, wobei optional zur Regelung der Impedanz die HF-Spannung U als Stellgröße dienen kann.

**Claims**

1. Device for controlling a treatment process, comprising a treatment tool, in particular a HF surgical instrument, an impedance detection device, an energy source for introducing electrical power into a material to be treated by the treatment tool, a timer and a control device for controlling the energy source, wherein the control device is designed to control the energy source such that, during a first treatment phase, the power that is fed into the material to be treated is regulated so as to have an increasing, e.g. ramp-like, course, and wherein there is a switch from power control to voltage control or impedance control upon fulfilment of an integral criterion.

2. Device according to claim 1, which is designed to limit the temporal voltage increase.

3. Device according to either claim 1 or claim 2, wherein the impedance detection device is designed to determine the impedance course and/or the present impedance of the material to be treated, and to detect when an impedance minimum has been reached, wherein the timer is started at regular or irregular intervals and/or upon detection of an impedance minimum and is set to a specific time interval, wherein the timer is reset and starts again upon detection of an impedance decrease or of a first or further impedance minimum within the specific time interval, and wherein the timer generates a signal if no new impedance minimum is detected within the specific time interval and the time interval has elapsed, which signal prompts the control device to switch from power control to a constant output or to a power course having a different gradient.

4.  Device according to any one of the preceding claims, which is designed to change from voltage control back to power control if the impedance decreases again for a predetermined period of time and/or if a new impedance minimum is detected.

5.  Device according to any one of the preceding claims, wherein the integral criterion is a temporal integral of the impedance increase over a smallest measured impedance, and is switched from power control to voltage control if the temporal integral reaches a threshold, and therefore the integral criterion can react to very quick and also to slow impedance changes.

6.  Device according to any one of the preceding claims, which is designed to reduce the voltage that has just been applied by a specific value or percentage, for example by from 2 % to 70 % or from 5 % to 20 %, in the event of a switch from power control to voltage control.

7.  Device according to any one of the preceding claims, which is designed to measure the initial tissue impedance during a starting phase, preferably at a very low power.

8.  Device according to any one of the preceding claims, which is designed to use an integral criterion in order to switch to power stabilisation, wherein the temporal integral of the impedance increase over the smallest impedance measured until this point is determined and switched as soon as the temporal integral ($Zs(n)$) reaches a threshold.

9.  Device according to claim 8, which is designed to form a quotient from the temporal integral and the smallest impedance in order to achieve a normalisation to a relevant impedance level.

10. Device according to claim 8, which is designed to form a quotient from the temporal integral and the starting impedance in order to achieve a normalisation to a relevant impedance level.

11. Device according to any one of the preceding claims, comprising a digital and/or analogue filter, wherein the present tissue impedance is determined by filtering using the filter.

12. Device according to any one of the preceding claims, which is designed to carry out an impedance acceleration after a transition into a holding phase, wherein the impedance (Z) gradient is increased, e.g. linearly, it optionally being possible for the HF voltage U to be used as a manipulated variable for regulating the impedance.

**Revendications**

1.  Dispositif de commande d'une procédure de commande, avec un outil de traitement, en particulier un instrument chirurgical HF, d'un système de détection d'impédance, avec une source d'énergie pour introduire la puissance électrique dans un matériau à traiter par l'outil de traitement, une minuterie et un système de commande pour la commande de la source d'énergie,
dans lequel le système de commande est configuré pour commander la source d'énergie de manière que, dans une première phase de traitement, une régulation de la puissance fournie au matériau à traiter a lieu selon une courbe ascendante, par exemple en forme de rampe,
et dans lequel, en cas de satisfaction d'un critère intégral, il y a une commutation de la régulation de puissance à une régulation de tension ou à une régulation d'impédance.

2.  Dispositif selon la revendication 1, qui est configuré pour limiter la montée temporelle de tension.

3.  Dispositif selon la revendication 1 ou 2, dans lequel le système de détection d'impédance est configuré pour déterminer la courbe d'impédance et/ou l'impédance actuelle du matériau à traiter et à détecter la réalisation d'un minimum d'impédance,
dans lequel la minuterie est démarrée à des intervalles réguliers ou irréguliers et/ou est démarrée en cas de détection d'un minimum d'impédance et est réglée sur un intervalle de temps déterminé,
dans lequel, en cas de détection d'une diminution d'impédance ou d'un premier ou d'un autre minimum d'impédance à l'intérieur de l'intervalle de temps déterminé, la minuterie est remise à zéro et commence de nouveau à fonctionner, et dans lequel la minuterie ensuite, lorsqu'aucun nouveau minimum d'impédance n'est détecté à l'intérieur de l'intervalle de temps déterminé et que l'intervalle de temps expire, génère un signal qui amène le système de commande à commuter la régulation de puissance à une puissance constante ou à une courbe de puissance avec une pente modifiée.

4. Dispositif selon l'une quelconque des revendications précédentes, qui est configuré pour passer de la régulation de tension de nouveau à la régulation de puissance lorsque l'impédance chute de nouveau pendant un temps prédéfini et/ou qu'un nouveau minimum d'impédance est détecté.

5. Dispositif selon l'une quelconque des revendications précédentes, où le critère intégral est une intégrale par rapport au temps sur l'élévation d'impédance par rapport à une impédance la plus petite mesurée, et il y a une commutation de la régulation de puissance à la régulation de tension lorsque l'intégrale par rapport au temps atteint une valeur seuil de sorte que le critère intégral puisse réagir à des transitions d'impédance ou à des modifications d'impédance très rapides mais aussi très lentes.

6. Dispositif selon l'une quelconque des revendications précédentes, qui est configuré pour, en cas de commutation d'une régulation de puissance à une régulation de tension, diminuer la tension justement appliquée d'une valeur ou d'un pourcentage déterminé, par exemple, de 2 à 70 % ou de 5 % à 20 %.

7. Dispositif selon l'une quelconque des revendications précédentes, qui est configuré pour mesurer, dans une phase initiale, l'impédance initiale du tissu, de préférence avec une très petite puissance.

8. Dispositif selon l'une quelconque des revendications précédentes, qui est configuré pour employer un critère intégral pour la commutation au maintien constant de la puissance ; dans lequel l'intégrale par rapport au temps sur l'élévation d'impédance par rapport à la plus petite impédance, qui a été mesurée jusqu'à présent, est déterminée et est commutée dès que l'intégrale par rapport au temps (Zs(n)) atteint une valeur seuil.

9. Dispositif selon la revendication 8, qui est configuré pour former un quotient de l'intégrale par rapport au temps et de la plus petite impédance afin d'atteindre une normalisation sur un niveau d'impédance concerné.

10. Dispositif selon la revendication 8, qui est configuré pour former un quotient de l'intégrale par rapport au temps et de l'impédance de départ pour atteindre une normalisation sur un niveau d'impédance concerné.

11. Dispositif selon l'une quelconque des revendications précédentes, avec un filtre numérique et/ou analogique, dans lequel l'impédance actuelle du tissu est déterminée par filtrage au moyen du filtre.

12. Dispositif selon l'une quelconque des revendications précédentes, qui est configuré pour exécuter, après un passage dans une phase de maintien, une accélération d'impédance au cours de laquelle la pente de l'impédance (Z) est augmentée, par exemple linéairement, dans lequel, de manière facultative, la tension HF U peut servir de grandeur de réglage pour la régulation de l'impédance.

Fig.1

Fig.2

Aussetzen des Leistungsanstiegs während der Erwärmungsphase

Fig.3

Exemplarischer Ablauf des TFT Prozesses

Fig.4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6733498 B2
- EP 2025297 A2

- US 2011160725 A